(19) Europäisches Patentamt
European Patent Office
Office européen des brevets

(11) Numéro de publication : **0 149 952**
**B1**

(12) **FASCICULE DE BREVET EUROPÉEN**

(45) Date de publication du fascicule du brevet : **08.07.87**

(51) Int. Cl.⁴ : **C 07 C 45/63**, C 07 C 47/565, C 07 C 47/575

(21) Numéro de dépôt : **84420212.7**

(22) Date de dépôt : **19.12.84**

(54) **Procédé de préparation de bromobenzaldéhydes hydroxy et/ou alkoxy substitués.**

(30) Priorité : **22.12.83 FR 8320798**

(43) Date de publication de la demande : **31.07.85 Bulletin 85/31**

(45) Mention de la délivrance du brevet : **08.07.87 Bulletin 87/28**

(84) Etats contractants désignés : **AT BE CH DE GB IT LI LU NL SE**

(56) Documents cités :
**FR-A- 2 187 735**
**GB-A- 1 338 890**
**Le dossier contient des informations techniques présentées postérieurement au dépôt de la demande et ne figurant pas dans le présent fascicule.**

(73) Titulaire : **RHONE-POULENC SPECIALITES CHIMIQUES**
**"Les Miroirs" 18, Avenue d'Alsace**
**F-92400 Courbevoie (FR)**

(72) Inventeur : **Ratton, Serge**
**Hameau de Belmont Vaulx-Milieu**
**F-38090 - Villefontaine (FR)**
Inventeur : **Bougeois, Jean-Luc**
**4, Chemin du Signal**
**F-69110 Sainte-Foy-lès-Lyon (FR)**

(74) Mandataire : **Vignally, Noel et al**
**RHONE-POULENC INTERSERVICES Service Brevets Chimie Centre de Recherches de Saint-Fons B.P. 62**
**F-69192 Saint-Fons Cedex (FR)**

**EP 0 149 952 B1**

Il est rappelé que : Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

Jouve, 18, rue St-Denis, 75001 Paris, France

## Description

La présente invention a pour objet un procédé de préparation de bromobenzaldéhydes comportant des substituants hydroxy et/ou alkoxy et plus particulièrement de la bromo-5 vanilline.

Les bromobenzaldéhydes comportant des substituants hydroxy et/ou alkoxy sont des produits industriels précieux utilisés comme intermédiaires en synthèse organique. Ainsi la bromo-5 vanilline (bromo-3 hydroxy-4 méthoxy-5 benzaldéhyde), l'aldéhyde bromoprotocatéchique (bromo-3 dihydroxy-4,5 benzaldéhyde) et le bromo-3 diméthoxy-4,5 benzaldéhyde sont utilisés comme intermédiaires pour la préparation du triméthoxy-3,4,5 benzaldéhyde qui intervient lui-même dans la fabrication de produits pharmaceutiques tels que le triméthoprim [diamino-2,4 (triméthoxy-3,4,5 benzyl)-5 pyrimidine]. Ces bromobenzaldéhydes sont également utilisés pour la préparation de bromophénylalanines possédant une activité comme hypotenseur (cf. brevet français 1 592 518).

Les bromobenzaldéhydes alkoxy et/ou hydroxy substitués sont préparés par réaction du brome avec l'aldéhyde correspondant.

On connaît divers procédés de bromation des aldéhydes aromatiques. Ainsi on a proposé de conduire la bromation des hydroxy et/ou alcoxybenzaldéhydes dans divers milieux réactionnels. Le solvant le plus couramment utilisé est l'acide acétique glacial contenant éventuellement un acétate alcalin comme l'acétate de sodium, cf. DAKIN, Am. Chem. Journal 42 477-98 (1909) ; TORREY et al, J. Am. Chem. Soc. 31 583-585 (1909) ; O.S. BRADY et al, J. Chem. Soc. 107 1858-62 (1915) ; E.I. SHRINER et al, J. Am. Chem. Soc. 51 2194 (1929) ; R.A. Mc IVOR et al, Cand. J. of Chem. 32 298-302 (1953) ; HENRY et al, J. Chem. Soc. (1930) 2279-89 ; F. MISANI et al, J. Org. Chem. 10 356 (1945) ; R. PSCHORR Ann. 391 23-39 (1912) ; brevet français 1 592 518. Bien que ce procédé conduise à d'excellents rendements en bromobenzaldéhydes, notamment dans le cas de la vanilline, il souffre de divers inconvénients qui le rende peu attrayant au plan industriel. En particulier ce procédé conduit en fin de réaction à une solution d'acide bromhydrique dans l'acide acétique à partir de laquelle il est difficile, voire pratiquement impossible, de récupérer HBr.

On a encore proposé (cf. R. PSCHORR loc. cit.) de remplacer l'acide acétique glacial par le chloroforme ; dans ce cas il est difficile de débarrasser le bromobenzaldéhyde de l'acide bromhydrique qu'il contient par lavage au chloroforme ce qui implique le recours à un tiers solvant de lavage et rend le procédé complexe à mettre en œuvre industriellement.

Dans le brevet français 72/38.410, publié sous le No. 2 177 693, on a décrit un procédé de bromation de la vanilline consistant à ajouter une solution de vanilline dans l'acide bromhydrique à 48 % en poids d'HBr à du brome.

Les alcools inférieurs et notamment l'éthanol, ont été également utilisés comme milieu de bromation (cf. F. TIEMANN et al, Ber 7 615 [1874]). La formation conjointe de bromure de méthyle irrécupérable ou de bromure d'éthyle qui peut être difficile à valoriser dans le cadre d'une production importante de bromovanilline rend ce procédé peu attractif.

Dans tous les cas la réaction aboutit à la formation d'une molécule d'acide bromhydrique par molécule de bromobenzaldéhydes produit conformément au schéma réactionnel suivant :

a) 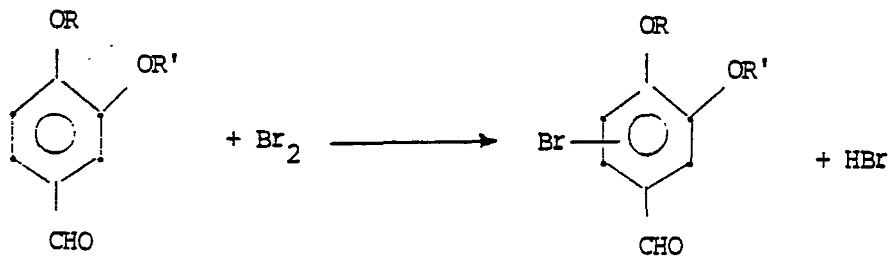

On constate que dans un tel processus seulement la moitié du brome engagé est utilisée pour la formation des bromobenzaldéhydes, l'autre moitié engendrant de l'acide bromhydrique où, selon le solvant utilisé, des bromures d'alkyle. La récupération et/ou la valorisation de ces sous-produits diminuent l'intérêt industriel de ce procédé quel que soit son mode de mise en œuvre.

La demande de brevet FR-A-2 187 735 décrit un procédé de dihalogénation de composés aromatiques au moyen de couples halogène/eau oxygénée ; une première halogénation est effectuée à l'aide d'une quantité stœchiométrique d'halogène, puis une deuxième halogénation est réalisée par le couple acide halohydrique formé/eau oxygénée.

Ce procédé ne résoud pas le problème de la monobromation d'un benzaldéhyde substitué, sans perte de brome sous forme d'acide bromhydrique.

Ainsi il ressort de cette analyse de l'état de la technique que la formation conjointe d'HBr résultant de l'emploi du brome comme agent de bromation pose un problème pour la mise en œuvre industrielle des procédés connus.

La présente invention a précisément pour objet de résoudre le problème posé par la formation de

l'acide bromhydrique engendré au cours de la réaction de bromation.

Plus spécifiquement la présente invention se rapporte à un procédé de préparation de bromobenzaldéhydes substitués de formule générale :

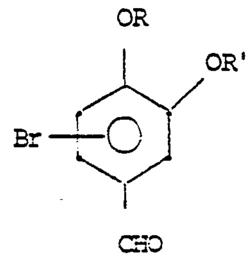

(I)

dans laquelle R et R', identiques ou différents, représentent un atome d'hydrogène ou un radical méthyle ou éthyle par réaction d'un aldéhyde formule générale :

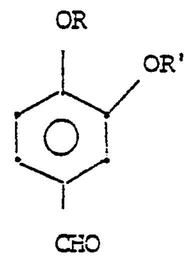

(II)

avec du brome, caractérisé en ce que l'on opère avec une quantité de brome inférieure à la stœchiométrie de la réaction et que l'on achève la réaction de bromation par l'intervention du couple constitué par l'acide bromhydrique engendré dans la réaction et un agent oxydant des ions bromures.

Comme agent oxydant des ions bromures on peut faire appel à n'importe quel composé chimique connu comme ayant cette propriété. On peut faire appel notamment à l'eau oxygénée, à l'acide nitrique, aux ions hypochlorites en particulier sous forme d'hypochlorites alcalins.

Bien qu'il soit, en général, connu d'oxyder les ions bromures en brome à l'aide de certains oxydants l'emploi du couple HBr/oxydant pour réaliser la bromation des hydroxy et/ou alcoxy benzaldéhydes pouvaient laisser craindre le déroulement de réactions d'oxydation et/ou de substitution du produit de départ. Ainsi l'art antérieur enseigne l'oxydation par l'eau oxygénée du groupe aldéhyde selon une réaction du type Baeyer et Williger (cf. C.H. HASSAL, Organic Reactions, volume 9 pages 73 à 106 [1957] ; J.E. LEFFLER, Chem. Rev. 45 pages 385 à 410 [1949]). La solution du problème posé par la bromation des aldéhydes aromatiques hydroxy et/ou alkoxy substitués ne s'imposait donc pas de façon évidente.

Sans limiter en quoi que ce soit l'invention à un mécanisme particulier, on peut considérer que le procédé de bromation proposé ci-avant met en œuvre les réactions suivantes :

a) bromation partielle de l'aldéhyde par un défaut de brome selon le schéma :

i) 2 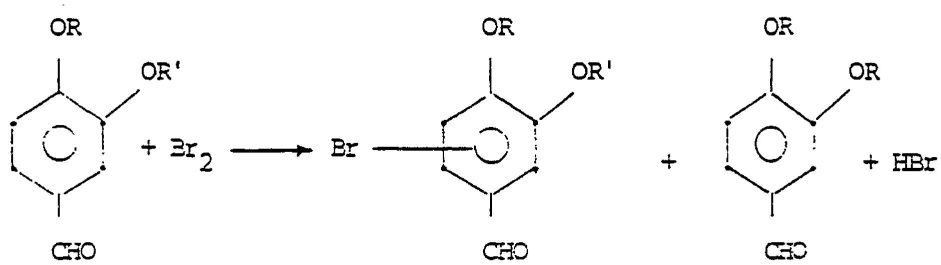

b) bromation de l'aldéhyde non transformé par le couple HBr/oxydant ; ainsi dans le cas où ce dernier est l'eau oxygénée la réaction peut être représentée par le schéma :

ii) 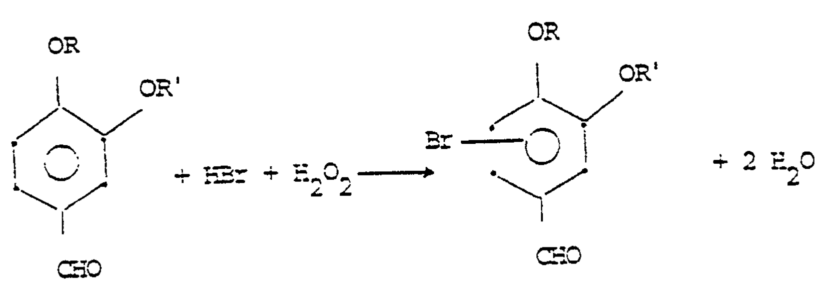

Le bilan global de la réaction peut alors être représenté par le schéma :

iii) 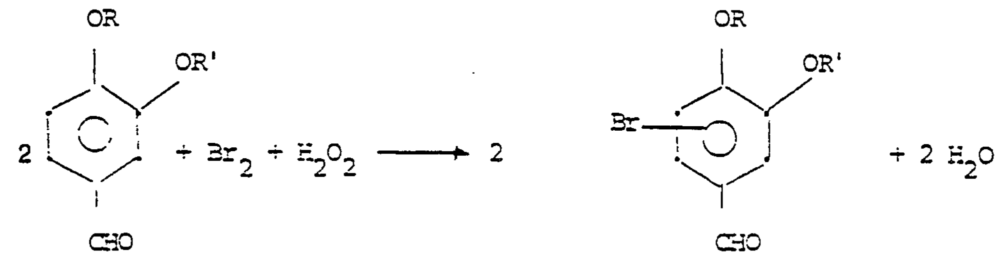

Le procédé selon l'invention permet donc de bromer les benzaldéhydes substitués par le brome et de récupérer directement à la bromation le brome présent dans l'acide bromhydrique sous produit.

Bien que la bromation selon l'invention puisse être conduite dans l'eau et/ou un solvant organique inerte de préférence non miscible à l'eau tels que les hydrocarbures aliphatiques halogénés ou les éthers, il est préférable, pour obtenir les taux de transformation et les rendements les plus élevés d'opérer en présence d'un acide aliphatique ou minéral inerte vis-à-vis du brome ou de l'oxydant. Dans ce cas la quantité d'acide exprimée en équivalent molaire par mole de benzaldéhyde est de préférence au moins égale à 0,001 équivalent par mole de benzaldéhyde et plus particulièrement à 0,01 équivalent par mole de benzaldéhyde. Il n'y a pas de limite supérieure critique de la quantité d'acide, celui-ci pouvant constituer le milieu réactionnel.

Selon une première variante, le procédé selon l'invention est réalisé au sein d'un acide alcanoïque comportant de 2 à 7 atomes de carbone tels que les acides acétique, propionique, butyrique, n-pentanoïque, n-hexanoïque. On utilise de préférence l'acide acétique dans lequel les benzaldéhydes de départ sont solubles et qui peut constituer le milieu de réaction. On peut utiliser indifféremment une solution aqueuse d'acide dont la concentration n'est pas critique ou un acide anhydre.

Selon une seconde variante le procédé est réalisé en présence d'un acide minéral qui peut également constituer au moins partiellement le milieu réactionnel. On fait appel de préférence à des solutions aqueuses d'acides bromhydrique et sulfurique dont la concentration n'est pas critique et peut varier dans de larges limites. Ainsi on peut utiliser des solutions aqueuses d'acide sulfurique contenant de 5 à 65 % en poids de $H_2SO_4$ ou des solutions aqueuses d'acide bromhydrique contenant de 5 à 60 % en poids de HBr. Dans le dernier cas on utilise de préférence des solutions contenant de 45 à 55 % en poids de HBr car la solubilité des aldéhydes de départ augmente avec la concentration en HBr. Selon un mode préféré de réalisation de l'invention la solution aqueuse d'acide minéral constitue au moins partiellement le milieu réactionnel. Ainsi on peut procéder à la bromation sur une suspension de benzaldéhyde dans une solution aqueuse d'$H_2SO_4$ ou d'HBr ou sur une solution de benzaldéhydes dans une solution concentrée d'HBr. On peut également utiliser conjointement à la solution aqueuse acide un solvant organique du benzaldéhyde et du brome, inerte dans les conditions de la réaction, et de préférence non-miscible à l'eau ; on met alors en contact une phase organique contenant le benzaldéhyde avec une phase aqueuse acide au sein de laquelle les ions bromures sont oxydés. Il s'agit là d'un mode de réalisation préféré du procédé selon l'invention. La variante qui consiste à mettre en œuvre une solution aqueuse d'acide bromhydrique et un solvant organique convient tout particulièrement bien à la mise en œuvre du procédé. Parmi les solvants qui se prêtent à l'exécution de ce mode de réalisation on peut citer les hydrocarbures halogénés (chlorure de méthylène, chloroforme, tétrachlorure de carbone), les éthers aliphatiques (oxyde d'isopropyle, oxyde d'amyle, oxyde de butyle et d'éthyle, oxyde de t-butyle et d'éthyle, oxyde de n-butyle, oxyde de n-propyle, oxyde de n-butyle et de méthyle).

La concentration de l'aldéhyde aromatique de départ dans le milieu de réaction retenu n'est pas critique et peut varier dans de larges limites. Elle dépend principalement de considérations d'ordre pratique telle que l'agitabilité de la masse réactionnelle et la productivité du procédé.

Bien que la quantité de brome mise en œuvre dans le procédé selon l'invention, exprimée en mole par mole d'aldéhyde puisse quelque peu varier à la condition de rester inférieure à la quantité stœchiométrique résultant du schéma a), il est préférable que cette quantité reste voisine de la moitié de la quantité stoechiométrique si l'on veut éviter ou limiter le plus possible la perte de brome sous forme d'acide bromhydrique. Ainsi la quantité de brome peut être comprise de préférence entre 0,45 et 0,65 mole de brome par mole de benzaldéhyde. Naturellement on ne sortirait pas du cadre de la présente invention en sortant quelque peu des limites de cet intervalle. L'emploi d'une quantité de brome inférieure à 0,45 mole par mole de benzaldéhyde se traduirait cependant par une transformation incomplète du produit de départ, et le recours à une quantité de brome supérieure à 0,65 mole par mole d'aldéhyde accroîtrait d'autant la formation de l'acide bromhydrique non récupérable à la bromation. On opère de préférence avec de 0,5 à 0,6 mole de brome par mole de benzaldéhyde.

La quantité d'agent oxydant mis en œuvre dépend évidemment de la quantité de brome utilisée et de la nature de l'oxydant. Il est préférable qu'elle soit en tout cas suffisante pour assurer la bromation complémentaire du benzaldéhyde formé par l'acide bromhydrique produit. La quantité d'oxydant sera exprimée par la suite en mole par mole de brome puisque la quantité d'acide bromhydrique formée dépend de la quantité de brome mise en œuvre.

Lorsque l'agent oxydant est l'eau oxygénée la quantité de $H_2O_2$ est de préférence voisine de la quantité stœchiométrique illustrée par le schéma réactionnel III), c'est-à-dire voisine de 1 mole par mole de brome. On pourrait s'écarter quelque peu de cette quantité sans sortir du cadre de la présente invention.

En pratique cette quantité est de préférence comprise entre 0,8 et 1,2 mole par mole de brome et dépend dans une certaine mesure de la nature du milieu réactionnel et de l'acide utilisé. Lorsqu'on utilise comme acide une solution aqueuse d'acide bromhydrique on peut utiliser un léger excès d'eau oxygénée. Dans les autres cas, il est préférable de ne pas dépasser 1 mole d'$H_2O_2$ par mole de brome et même d'opérer avec un léger défaut d'eau oxygénée.

La concentration de la solution aqueuse de $H_2O_2$ mise en œuvre n'est pas critique. Son choix est dicté par des considérations pratiques familières à l'homme de métier (par exemple souci de ne pas augmenter le volume de la masse réactionnelle). En général cette concentration peut aller de 20 à 90 % en poids de $H_2O_2$.

Quand on utilise un hypochlorite alcalin comme oxydant la réaction de bromation du benzaldéhyde en excès par le couple HBr/hypochlorite peut être représentée par le schéma :

iv) 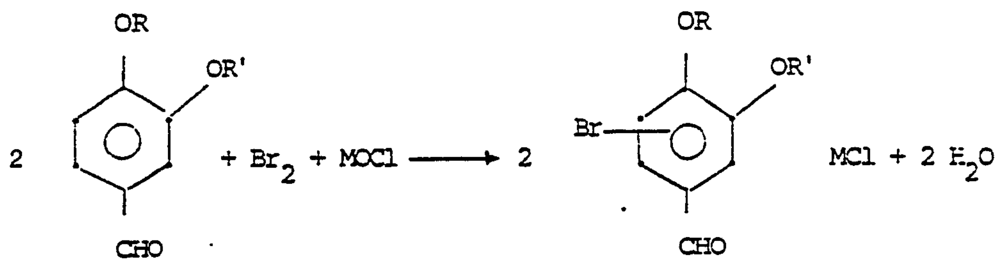


dans lequel M représente un métal alcalin. Dans ce cas la quantité d'hypochlorite est également de préférence voisine de la stoechiométrie de la réaction, c'est-à-dire de 1 mole par mole de brome. On a recours en pratique à des quantités d'hypochlorite allant de 0,7 à 1,1 mole par mole de brome et de préférence de 0,7 à 1 mole par mole de brome. La concentration des solutions aqueuses d'hypochlorite n'est pas critique.

Lorsqu'on fait appel à l'acide nitrique à titre d'oxydant la réaction est représentée par le schéma suivant :

v) 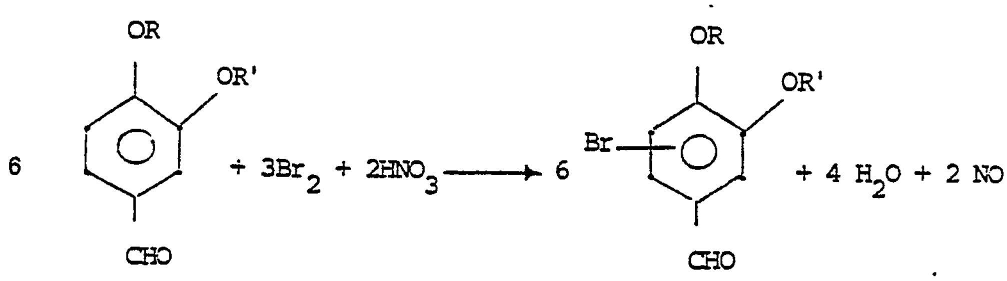


La quantité d'acide nitrique utilisée pour assurer l'oxydation des bromures en brome est de préférence voisine de la stoechiométrie de la réaction représentée par le schéma v) c'est-à-dire voisine de 2/3 de mole de $HNO_3$ pour 1 mole de $Br_2$. On peut cependant s'écarter sensiblement de la stoechiométrie sans pour autant sortir du cadre de l'invention. Ainsi la quantité d'acide nitrique peut varier à l'intérieur d'un intervalle de 0,3 à 0,8 mole de $HNO_3$ par mole de brome ou, de préférence, de 0,6 à 0,7 mole de $HNO_3$ par mole de brome.

La concentration de la solution aqueuse d'acide nitrique utilisée pour achever la bromation n'est pas critique et peut être comprise entre 20 et 90 % en poids de $HNO_3$. On a toutefois intérêt à utiliser des solutions concentrées pour ne pas augmenter le volume de la masse réactionnelle. Des solutions contenant de 55 à 70 % en poids de $HNO_3$ conviennent bien.

On a constaté qu'il est avantageux d'utiliser conjointement à l'acide nitrique une petite quantité d'acide nitreux qui assure un démarrage rapide de la réaction. Dans ce cas on a recours à un nitrite alcalin ($NaNO_2$ ; $KNO_2$) comme initiateur. Une quantité de l'ordre de 0,01 mole de nitrite par mole d'aldéhyde est suffisante pour initier la réaction ; en général il n'est pas nécessaire d'utiliser plus de 0,2 mole de nitrite par mole de benzaldéhyde ; des quantités comprises entre 0,05 et 0,15 mole de nitrite par mole de benzaldéhyde conviennent bien.

La température à laquelle on conduit la réaction de bromation peut être comprise entre 0 et 100 °C et de préférence entre 5 et 60 °C.

Parmi les aldéhydes de formule (I) qui peuvent être bromés par le procédé de l'invention on peut citer

l'aldéhyde protocatéchique (dihydroxy-3,4 benzaldéhyde), la vanilline, l'éthylvanilline, l'isovanilline et l'aldéhyde vératrique (diméthoxy-3,4 benzaldéhyde). L'aldéhyde protocatéchique, la vanilline et l'éthylvanilline conduisent aux bromobenzaldéhydes comportant un atome de brome en position méta par rapport au groupe aldéhyde. L'aldéhyde vératrique conduit au bromo-2 hydroxy-4 méthoxy-3 benzaldéhyde et l'isovanilline au bromo-2 méthoxy-4 hydroxy-5 benzaldéhyde). Le procédé selon l'invention convient tout spécialement bien à la bromation de la vanilline en bromo-5 vanilline.

Le présent procédé se prête particulièrement bien à une mise en œuvre continue.

Les exemples suivants illustrent l'invention et montrent comment elle peut être mise en pratique.

## Exemple 1

Dans un ballon en verre de 100 ml équipé d'un système d'agitation, d'un thermomètre, d'une ampoule de coulée et refroidi par un bain d'eau à 20 °C, on charge 20 ml d'acide acétique anhydre puis 7,5 g de vanilline (0,05 mole). On met l'agitation en marche puis, lorsque la vanilline est dissoute, on ajoute goutte à goutte une solution de 4,8 g de brome (0,03 mole) dans 10 ml d'acide acétique. La température s'élève progressivement à 30 °C. Lorsque l'addition du brome est achevée on ajoute 20 ml d'acide acétique puis, goutte à goutte, 2,26 g d'eau oxygénée à 30 % de $H_2O_2$ (0,02 mole). On maintient l'agitation pendant 10 mn après la fin de la coulée et refroidit la masse réactionnelle hétérogène à 20 °C. On la filtre et lave le gâteau sur filtre par 10 ml d'acide acétique frais puis 30 ml d'eau glacée. Après séchage sous vide on recueille 10,8 g d'un produit fondant à 162 °C dans lequel on dose 10,60 g de bromo-5 vanilline par chromatographie liquide sous haute pression. On dose dans le filtrat et l'acide acétique de lavage 0,45 g de vanilline non transformée.

Le taux de conversion de la vanilline s'élève à 94 % et le rendement en bromo-5 vanilline par rapport à la vanilline transformée à 98 %.

## Exemples 2 à 3

On opère comme à l'exemple 1 dans 50 ml d'acide acétique et en utilisant les rapports molaires vanilline/brome/$H_2O_2$ suivants :

| EXEMPLES | vanilline (moles) | $Br_2$ mole | $H_2O_2$ mole | TT (1) % | RT (2) % |
|---|---|---|---|---|---|
| 2 | 2 | 1 | 1,04 | 96,6 | 90 |
| 3 | 2 | 1,1 | 1 | 92,6 | 92,7 |

(1) taux de transformation de la vanilline
(2) rendement en bromovanilline par rapport à la vanilline transformée.

## Exemple 4

Dans un ballon de 250 ml équipé comme à l'exemple 1 on charge 100 ml d'une solution aqueuse 2N d'acide sulfurique puis 15,15 g de vanilline (0,1 mole). On met l'agitation en marche puis on ajoute à la suspension de vanilline ainsi obtenue 9,6 g (0,6 mole) de brome goutte à goutte ; la température s'élève progressivement à 30 °C. Lorsque l'addition est terminée on coule de la même manière 4,42 g d'une solution aqueuse à 30,8 % en poids d'$H_2O_2$ (soit 0,04 mole). Après la fin de la coulée on maintient encore

5 mn sous agitation. La masse réactionnelle hétérogène est refroidie à 20 °C puis la phase solide est séparée par filtration, lavée sur filtre par de l'eau puis séchée à 60 °C sous pression réduite. Le filtrat est extrait 3 fois par 150 ml de chlorure de méthylène. On dose la vanilline non transformée et la bromovanilline dans la phase solide et dans le chlorure de méthylène de lavage par chromatographie liquide sous haute pression.

On dose de cette façon 2,92 g de vanilline (soit un taux de transformation de 80,7 %) et 16,42 g de bromo-5 vanilline (0,071 mole), ce qui correspond à un rendement théorique de 88,1 % par rapport à la vanilline transformée.

## Exemple 5

Dans l'appareil de l'exemple 4 on charge 110 ml de chloroforme puis 15,15 g de vanilline et on met l'agitation en marche. Lorsque la vanilline est dissoute on ajoute 20 ml d'une solution aqueuse 2N de $H_2SO_4$. On introduit ensuite goutte à goutte dans la masse hétérogène ainsi obtenue 9,6 g de brome (0,06 mole). La température s'élève progressivement à 30 °C. Lorsque l'addition du brome est achevée on charge de la même façon 4,42 g d'une solution aqueuse à 30 % en poids de $H_2O_2$ (soit 0,04 mole). La bromo-5 vanilline qui a précipité au fur et à mesure de sa formation est séparée par filtration. Les phases liquides du filtrat sont séparées par décantation et la phase aqueuse est extraite 3 fois par 150 ml de chlorure de méthylène à chaque fois.

En utilisant la même méthode qu'aux exemples précédents on a dosé au total 1,21 g de vanilline non transformée (soit un taux de transformation de 92 %) et 21,07 g de bromo-5 vanilline, ce qui représente un rendement de 99,5 % par rapport à la vanilline transformée.

## Exemple 6

On a opéré dans les mêmes conditions qu'à l'exemple 5 mais en remplaçant l'acide sulfurique par 50 ml d'une solution aqueuse 2N d'acide bromhydrique, le volume de chloroforme étant de 100 ml.

Dans ces conditions le taux de transformation de la vanilline a été de 94,84 % et le rendement en bromo-5 vanilline par rapport à la vanilline transformée de 96,4 %.

## Exemple 7

On a opéré comme à l'exemple 6 mais en utilisant un rapport molaire vanilline/brome/$H_2O_2$ égal à 2/1/1,1.

Dans ces conditions le taux de transformation de la vanilline a été de 95,5 % et le rendement en bromo-5 vanilline par rapport à la vanilline transformée de 98,1 %.

## Exemple 8

On a opéré comme à l'exemple 7 en remplaçant l'acide bromhydrique 2N par 40 ml d'une solution aqueuse à 48 % en poids d'HBr.

Dans ces conditions le taux de transformation de la vanilline a été de 95,5 % et le rendement en bromo-5 vanilline par rapport à la vanilline transformée de 94,7 %.

## Exemple 9

On a répété l'exemple 7 mais en laissant la température s'élever à 50 °C en fin de réaction.

Dans ces conditions le taux de transformation de la vanilline a été de 94,8 % et le rendement en bromo-5 vanilline par rapport à la vanilline transformée de 95,7 %.

## Exemple 10

On a répété l'exemple 7 mais en baissant la température à 0 °C.

Dans ces conditions le taux de transformation de la vanilline a été de 82,26 % et le rendement en bromo-5 vanilline par rapport à la vanilline transformée de 96,6 %.

Exemple 11

On a répété l'exemple 7 en remplaçant l'eau oxygénée par 0,0187 mole d'$HNO_3$ (solution aqueuse à 65 % en poids de $HNO_3$) et 0,01 mole de nitrite de sodium.

Dans ces conditions le taux de transformation de la vanilline a été de 75,6 % et le rendement en bromo-5 vanilline par rapport à la vanilline transformée de 91,02 %.

Exemple 12

On a répété l'exemple 7 en remplaçant l'eau oxygénée par 0,027 7 mole de NaOCl (solution aqueuse 2N).

Dans ces conditions le taux de transformation de la vanilline a été de 76,8 % et le rendement en bromo-5 vanilline par rapport à la vanilline transformée de 89,2 %.

Exemple 13

On a opéré comme à l'exemple 7 mais en ajoutant simultanément le brome et l'eau oxygénée.

Dans ces conditions le taux de transformation de la vanilline a été de 97,4 % et le rendement en bromo-5 vanilline par rapport à la vanilline transformée de 94,1 %.

Exemple 14

On a répété l'exemple 7 après avoir remplacé le chloroforme par 250 ml d'oxyde d'isopropyle.

Dans ces conditions le taux de transformation de la vanilline a été de 86,2 % et le rendement en bromo-5 vanilline par rapport à la vanilline transformée de 94,1 %.

Exemple 15

On a opéré comme à l'exemple 7 mais en remplaçant la solution aqueuse 2N d'HBr par une solution aqueuse d'HBr 0,5 N (même volume : 50 ml).

Dans ces conditions on obtient un taux de transformation de la vanilline de 71,5 % et un rendement en bromo-5 vanilline par rapport à la vanilline transformée de 86,2 %.

Exemple 16

On a opéré comme à l'exemple 7 mais en remplaçant la solution aqueuse d'HBr 2N par l'eau seule.

On obtient un taux de transformation de la vanilline de 64,2 % et un rendement en bromo-5 vanilline par rapport à la vanilline de 82,9 %.

Exemple 17

On a répété l'exemple 11 mais en introduisant 0,050 mole de $HNO_3$ sous forme de solution aqueuse à 65 % et 0,01 mole de $NaNO_2$.

Dans ces conditions on obtient un taux de transformation de la vanilline de 96,7 % et un rendement en bromo-5 vanilline par rapport à la vanilline transformée de 83 %.

Exemple 18

On a répété l'exemple 13 mais en introduisant 0,050 mole de NaOCl (solution aqueuse 2N).

Dans ces conditions, le taux de transformation de la vanilline est de 81,1 % et le rendement en bromo-5 vanilline par rapport à la vanilline transformée est de 85,7 %.

**Revendications**

1. Procédé de préparation de bromobenzaldéhydes substitués de formule générale :

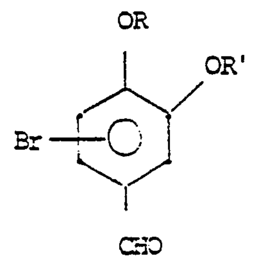

(I)

dans laquelle R et R', identiques ou différents, représentent un atome d'hydrogène ou un radical méthyle ou éthyle par réaction d'un aldéhyde formule générale :

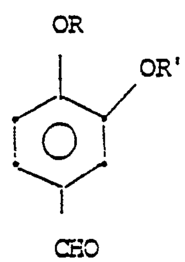

(II)

avec du brome, caractérisé en ce que l'on opère avec une quantité de brome inférieure à la stoechiométrie de la réaction et que l'on achève la réaction de bromation par l'intervention du couple constitué par l'acide bromhydrique engendré dans la réaction et un agent oxydant des ions bromures.

2. Procédé selon la revendication 1, caractérisé en ce que l'aldéhyde de formule (II) est mis en contact simultanément avec un défaut de brome et un oxydant.

3. Procédé selon la revendication 1, caractérisé en ce que la réaction est conduite en deux étapes successives consistant à bromer partiellement par le brome un benzaldéhyde de formule (II) puis à achever la bromation par addition d'un oxydant des ions bromures.

4. Procédé selon l'une quelconque des revendications 1 à 3, caractérisé en ce que l'oxydant est choisi dans le groupe constitué par l'eau oxygénée, l'acide nitrique et les hypochlorites alcalins.

5. Procédé selon l'une quelconque des revendications 1 à 4, caractérisé en ce que la quantité de brome est comprise entre 0,45 et 0,65 mole par mole de benzaldéhyde.

6. Procédé selon l'une quelconque des revendications 1 à 4, caractérisé en ce que la quantité d'eau oxygénée est comprise entre 0,8 et 1,2 mole par mole de brome.

7. Procédé selon l'une quelconque des revendications 1 à 4, caractérisé en ce que la quantité d'acide nitrique est comprise entre 0,3 et 0,8 mole de $HNO_3$ par mole de brome.

8. Procédé selon l'une quelconque des revendications 1 à 5, caractérisé en ce que la quantité d'hypochlorite est comprise entre 0,7 et 1,1 mole par mole de brome.

9. Procédé selon l'une quelconque des revendications 1 à 6, caractérisé en ce que l'on conduit la bromation en présence d'un acide carboxylique ou minéral.

10. Procédé selon l'une quelconque des revendications 1 à 9, caractérisé en ce que la réaction est conduite dans un acide alcanoïque liquide dans les conditions de la réaction.

11. Procédé selon la revendication 10, caractérisé en ce que l'acide alcanoïque est l'acide acétique.

12. Procédé selon l'une quelconque des revendications 1 à 9, caractérisé en ce que la réaction est conduite dans une solution aqueuse d'un acide minéral pris dans le groupe formé par l'acide sulfurique et l'acide bromhydrique.

13. Procédé selon l'une quelconque des revendications 1 à 9, caractérisé en ce que la réaction est conduite dans un solvant organique inerte du benzaldéhyde et du brome, non miscible à l'eau et en présence d'une solution aqueuse d'un acide minéral pris dans le groupe formé par l'acide sulfurique et l'acide bromhydrique.

14. Procédé de préparation de bromobenzaldéhydes de formule (I) comportant un atome de brome en position méta par rapport au groupe aldéhyde selon l'une quelconque des revendications 1 à 12,

caractérisé en ce que l'on utilise comme aldéhyde de départ un aldéhyde de formule (II) dans laquelle R représente un atome d'hydrogène et R' un atome d'hydrogène ou un radical méthyle ou éthyle.

## Claims

1. Process for preparing substituted bromobenzaldehydes of general formula :

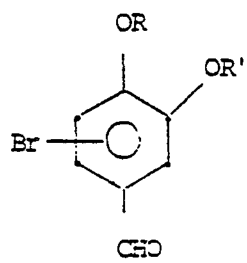

(I)

in which R and R', which are identical or different, denote a hydrogen atom or a methyl or ethyl radical, by reaction of an aldehyde of general formula :

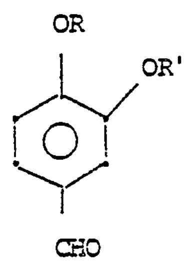

(II)

with bromine, which is carried out with a quantity of bromine which is smaller than the stoichiometry of the reaction and in which the bromination reaction is completed by the use of the pair consisting of the hydrobromic acid generated in the reaction and an oxidizer of bromide ions.

2. Process according to claim 1, in which the aldehyde of formula (II) is placed in contact simultaneously with a deficiency of bromine and an oxidizer.

3. Process according to claim 1, in which the reaction is conducted in two successive stages consisting in partially brominating with bromine a benzaldehyde of formula (II) and then completing the bromination by addition of an oxidizer of bromide ions.

4. Process according to any one of claims 1 to 3, in which the oxidizer is chosen from the group consisting of hydrogen peroxide, nitric acid and alkali metal hypochlorites.

5. Process according to any one of claims 1 to 4, in which the quantity of bromine is between 0.45 and 0.65 mole per mole of benzaldehyde.

6. Process according to any one of claims 1 to 4, in which the quantity of hydrogen peroxide is between 0.8 and 1.2 mole per mole of bromine.

7. Process according to any one of claims 1 to 4, in which the quantity of nitric acid is between 0.3 and 0.8 mole of $HNO_3$ per mole of bromine.

8. Process according to any one of claims 1 to 5, in which the quantity of hypochlorite is between 0.7 and 1.1 mole per mole of bromine.

9. Process according to any one of claims 1 to 6, in which the bromination is conducted in the presence of a carboxylic or inorganic acid.

10. Process according to any one of claims 1 to 9, in which the reaction is conducted in an alkanoic acid which is liquid under the reaction conditions.

11. Process according to claim 10, in which the alkanoic acid is acetic acid.

12. Process according to any one of claims 1 to 9, in which the reaction is conducted in an aqueous solution of an inorganic acid taken from the group consisting of sulphuric acid and hydrobromic acid.

13. Process according to any one of claims 1 to 9, in which the reaction is conducted in an inert, water-immiscible organic solvent for benzaldehyde and for bromine, and in the presence of an aqueous solution of an inorganic acid taken from the group consisting of sulphuric acid and hydrobromic acid.

14. Process for preparing bromobenzaldehydes of formula (I) containing a bromine atom in a meta position relative to the aldehyde group according to any one of claims 1 to 12, in which the starting aldehyde employed is an aldehyde of formula (II) in which R denotes a hydrogen atom and R' a hydrogen atom or a methyl or ethyl radical.

**Patentansprüche**

1. Verfahren zur Herstellung von substituierten Brombenzaldehyden der allgemeinen Formel

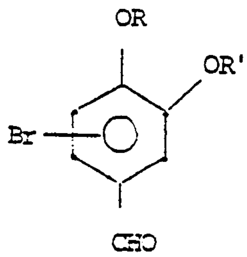

(I)

worin R und R', die identisch oder verschieden sind, ein Wasserstoffatom oder einen Methyl- oder Ethylrest bedeuten, durch Reaktion eines Aldehyds der allgemeinen Formel

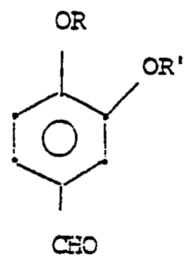

(II)

mit Brom, dadurch gekennzeichnet, daß man mit einer Brommenge unterhalb der Stöchiometrie der Reaktion arbeitet und daß man die Bromierungsreaktion mittels des Paares, bestehend aus bei der Reaktion erzeugter Bromwasserstoffsäure und einem Oxidationsmittel für Bromidionen, beendet.

2. Verfahren gemäß Anspruch 1, dadurch gekennzeichnet, daß der Aldehyd der Formel (II) gleichzeitig mit einem Bromunterschuß und einem Oxidationsmittel in Kontakt gebracht wird.

3. Verfahren gemäß Anspruch 1, dadurch gekennzeichnet, daß die Reaktion in zwei aufeinanderfolgenden Schritten durchgeführt wird, die darin bestehen, einen Benzaldehyd der Formel (II) teilweise mit Brom zu bromieren und dann die Bromierung durch Zugabe eines Oxidationsmittels für Bromidionen zu vollenden.

4. Verfahren gemäß einem der Ansprüche 1 bis 3, dadurch gekennzeichnet, daß das Oxidationsmittel ausgewählt ist aus der Gruppe, bestehend aus Wasserstoffperoxid, Salpetersäure und Alkalihypochloriten.

5. Verfahren gemäß einem der Ansprüche 1 bis 4, dadurch gekennzeichnet, daß die Brommenge zwischen 0,45 und 0,65 Mol pro Mol Benzaldehyd beträgt.

6. Verfahren gemäß einem der Ansprüche 1 bis 4, dadurch gekennzeichnet, daß die Wasserstoffperoxid-Menge zwischen 0,8 und 1,2 Mol pro Mol Brom beträgt.

7. Verfahren gemäß einem der Ansprüche 1 bis 4, dadurch gekennzeichnet, daß die Salpetersäure-Menge zwischen 0,3 und 0,8 Mol $HNO_3$ pro Mol Brom beträgt.

8. Verfahren gemäß einem der Ansprüche 1 bis 5, dadurch gekennzeichnet, daß die Hypochlorit-Menge zwischen 0,7 und 1,1 Mol pro Mol Brom beträgt.

9. Verfahren gemäß einem der Ansprüche 1 bis 6, dadurch gekennzeichnet, daß man die Bromierung in Gegenwart einer Carbon- oder Mineralsäure durchführt.

10. Verfahren gemäß einem der Ansprüche 1 bis 9, dadurch gekennzeichnet, daß die Reaktion in einer unter den Bedingungen der Reaktion flüssigen Alkansäure durchgeführt wird.

11. Verfahren gemäß Anspruch 10, dadurch gekennzeichnet, daß die Alkansäure Essigsäure ist.

12. Verfahren gemäß einem der Ansprüche 1 bis 9, dadurch gekennzeichnet, daß die Reaktion in einer wäßrigen Lösung einer Mineralsäure, genommen aus der Gruppe, gebildet durch Schwefelsäure und Bromwasserstoffsäure, durchgeführt wird.

13. Verfahren gemäß einem der Ansprüche 1 bis 9, dadurch gekennzeichnet, daß die Reaktion in einem organischen Lösungsmittel, das für den Benzaldehyd und Brom inert ist und mit Wasser nicht mischbar ist, und in Gegenwart einer wäßrigen Lösung einer Mineralsäure, genommen aus der Gruppe, gebildet durch Schwefelsäure und Bromwasserstoffsäure, durchgeführt wird.

14. Verfahren zur Herstellung von Brombenzaldehyden der Formel (I) mit einem Bromatom in meta-Stellung in bezug auf die Aldehydgruppe gemäß einem der Ansprüche 1 bis 12, dadurch gekennzeichnet, daß man als Ausgangs-Aldehyd einen Aldehyd der Formel (II) verwendet, worin R ein Wasserstoffatom und R' ein Wasserstoffatom oder einen Methyl- oder Ethylrest darstellt.